# EUROPÄISCHE PATENTANMELDUNG

(11) **EP 4 523 710 A1**
(43) Veröffentlichungstag der Anmeldung: **19.03.2025**
(21) Anmeldenummer: 24193688.9
(22) Anmeldetag: 08.08.2024
(51) Int. Cl.: A61L 2/07

(54) **PHARMAZEUTISCHE ANLAGE UND VERFAHREN ZUM BETREIBEN EINER PHARMAZEUTISCHEN ANLAGE**

(30) Priorität: 24.08.2023 DE 102023122778
(71) Anmelder: Syntegon Technology GmbH, 71332 Waiblingen (DE)
(72) Erfinder: Kosian, Thomas, 91610 Insingen (DE); Donaubauer, Theresa, 74585 Rot am See (DE)
(74) Vertreter: DREISS Patentanwälte PartG mbB

(57) **Zusammenfassung**

Die Erfindung betrifft eine pharmazeutische Anlage (10) zum Abfüllen von Pharmazeutika umfassend ein erstes Modul (12) mit einem gegenüber seiner Umgebung abgeschlossenen ersten Arbeitsraum (14), ein zweites Modul (16) mit einem gegenüber seiner Umgebung abgeschlossenen zweiten Arbeitsraum (18), mindestens eine Wandung (20) zur räumlichen Trennung der Arbeitsräume (14, 18), mindestens eine Schnittstelle (22) mit mindestens einer in der Wandung (20) angeordneten Öffnung (24) zum Verbinden der beiden Arbeitsräume (14, 18), wobei die Schnittstelle (22) einen Deckel (26) zum Verschließen und Freigeben der Öffnung (24) aufweist, wobei der Deckel (26) in einer Offenstellung die Öffnung (24) freigibt und in einer Geschlossenstellung die Öffnung (24) verschließt, wobei die Schnittstelle (22) einen lösbar mit der Wandung (20) verbindbaren Rahmen (28) aufweist, wobei der Deckel (26) mittels des Rahmens (28) an der Wandung (20) befestigbar ausgebildet ist. Die Erfindung betrifft zudem ein Verfahren zum Betreiben einer pharmazeutischen Anlage (10).

## Beschreibung

Die Erfindung betrifft eine pharmazeutische Anlage mit den Merkmalen des Oberbegriffs des Anspruchs 1 und ein Verfahren zum Betreiben einer pharmazeutischen Anlage mit Merkmalen des Anspruchs 11.

Pharmazeutische Anlagen müssen regelmäßig dekontaminiert werden. Ziel der Dekontamination ist insbesondere die Abtötung mikrobieller Organismen auf allen Oberflächen innerhalb der Anlage. Hierfür ist es notwendig, dass das Dekontaminationsmittel alle Oberflächen erreicht. Pharmazeutische Anlagen mit mindestens zwei Modulen weisen in der Regel eine Schnittstelle auf, die die beiden Module über eine Öffnung miteinander verbindet.

Die Öffnung kann mittels eines Deckels verschlossen werden. Der Deckel kann zwei aufblasbare Dichtungen zur Abdichtung der Öffnung bzw. der Schnittstelle aufweisen. Die Dekontamination der beiden Module erfolgt in der Regel nacheinander. Dabei wird jeweils die weiter von dem Modul, das dekontaminiert wird, entfernte Dichtung aufgeblasen, sodass die Abdichtung des Moduls, das dekontaminiert wird, gegeben ist. Aufgrund der nacheinander erfolgten Dekontamination der Module und die Umschaltung der doppelten Dichtung, kann gewährleistet werden, dass ein Spalt zwischen den beiden Dichtungen bzw. zwischen der Öffnung und dem Deckel nacheinander von beiden Seiten mit Dekontaminationsmittel beaufschlagt werden kann. So kann eine Benetzung aller Flächen des Deckels und damit der Schnittstelle gewährleistet werden.

Nachteilig dabei ist, dass die beiden Module nacheinander dekontaminiert werden müssen. Dies führt zu einer längeren Vorbereitungs- bzw. Dekontaminationszeit. Zudem sind der Deckel bzw. die beiden aufblasbaren Dichtungen aufwendig zu reinigen. Insbesondere bei Verarbeitung von hochpotenten Produkten kann dies zu Risiken bei der Handhabung des Deckels führen. Zudem weist der Deckel bedingt durch den Biegeradius der aufblasbaren Dichtungen eine runde bzw. ovale Form auf. Dies schränkt die geometrische Ausgestaltung des Deckels stark ein.

Es ist daher Aufgabe der vorliegenden Erfindung eine pharmazeutische Anlage und ein Verfahren zum Betreiben einer pharmazeutischen Anlage bereitzustellen, wobei die obigen Nachteile ausgeräumt werden.

Die obige Aufgabe wird durch eine pharmazeutische Anlage mit den Merkmalen des Anspruchs 1 gelöst. Die pharmazeutische Anlage kann zum, insbesondere aseptischen, Abfüllen von Pharmazeutika eingerichtet sein. Die pharmazeutische Anlage kann als eine Abfüllanlage ausgebildet sein.

Die pharmazeutische Anlage umfasst ein erstes Modul. Das erste Modul weist einen gegenüber seiner Umgebung abgeschlossenen ersten Arbeitsraum auf. Die pharmazeutische Anlage umfasst ein zweites Modul. Das zweite Modul weist einen gegenüber seiner Umgebung abgeschlossenen zweiten Arbeitsraum auf. Die pharmazeutische Anlage, das erste Modul und/oder das zweite Modul können als ein Reinraum ausgebildet sein. Die pharmazeutische Anlage kann drei oder mehr Module umfassen.

Ein Reinraum im vorliegenden Sinne kann ein Barrieresystem, z. B. RABS (Reduced Access Barrier System), ein Isolator (z. B. aseptischer oder zytotoxischer Isolator) oder auch ein Biosicherheitsschrank (Biosafety cabinet) sein. Ein Reinraum in diesem Sinne kann unter Überdruck stehen, um das Eindringen von Kontaminationen zu verhindern bzw. das Risiko eines Eindringens zu minimieren. Ein Reinraum in diesem Sinne kann alternativ auch unter einem Unterdruck betrieben werden, um das Entweichen etwaiger im Reinraum zu handhabender toxischer oder in sonstiger Weise gefährlicher Stoffe zu verhindern.

Die pharmazeutische Anlage umfasst mindestens eine Wandung zur räumlichen Trennung der Arbeitsräume der beiden Module. Die Wandung kann als Trennwand zwischen den beiden Modulen ausgebildet sein. Es ist denkbar, dass die Wandung einwandig oder mehrwandig ausgebildet und zwischen den beiden Modulen angeordnet sein kann.

Die pharmazeutische Anlage umfasst weiter mindestens eine Schnittstelle mit mindestens einer in der Wandung angeordneten Öffnung zum (fluidischen) Verbinden (bzw. Koppeln) des ersten Arbeitsraums mit dem zweiten Arbeitsraum (und umgekehrt). Die beiden Arbeitsräume der beiden Module können durch die Öffnung in eine Fluidkommunikation gebracht werden. Die Öffnung kann eine fluidische Verbindung bzw. Kopplung zwischen den beiden Arbeitsräumen bzw. den beiden Modulen darstellen. Die Öffnung kann einem Durchtransport von Packmittel (in die die Pharmazeutika abgefüllt werden können) von dem ersten Modul in das zweite Modul und/oder umgekehrt dienen. Die pharmazeutische Anlage kann drei oder mehr Module umfassen, wobei jeweils zwischen zwei benachbarten Modulen mindestens eine Schnittstelle angeordnet sein kann.

Mit einer fluidischen Verbindung bzw. einer fluidischen Kopplung ist vorliegend gemeint, dass ein Gas und/oder eine Flüssigkeit (Fluid) zwischen zwei fluidisch gekoppelten Elementen bzw. zwischen zwei in fluidischer Verbindung stehender Elemente fließen kann. Vorliegend kann mit einem Fluid ein Gas und/oder eine Flüssigkeit gemeint sein.

Die Schnittstelle umfasst einen Deckel zum Verschließen und Freigeben der Öffnung. Der Deckel gibt die Öffnung in einer Offenstellung frei. Der Deckel verschließt in einer Geschlossenstellung die Öffnung. Insbesondere kann der Deckel zwischen der Offenstellung und der Geschlossenstellung (hin und her) bewegt werden. Insbesondere ist ein Austausch eines Fluid zwischen den beiden Arbeitsräumen nicht möglich, wenn der Deckel in der Geschlossenstellung angeordnet ist. Mit anderen Worten, in Geschlossenstellung kann der Deckel die Öffnung dicht verschließen. Insbesondere ist ein Austausch eines Fluids zwischen den beiden Arbeitsräumen möglich, wenn der Deckel in der Offenstellung angeordnet ist.

Die Schnittstelle umfasst einen lösbar mit der Wandung verbindbaren Rahmen. Der Rahmen kann reversibel an der Wandung fixiert (angebracht) werden. Der Rahmen kann an der Wandung angeordnet bzw. mit der Wandung verbunden werden. Der Rahmen kann von der Wandung getrennt (gelöst) bzw. von der Wandung abgenommen werden.

Der Deckel kann einen Griff zur Handhabung umfassen. Der Griff kann ergonomisch geformt sein. Der Rahmen und der Deckel können derart ausgebildet sein, dass mittels des Griffs der Deckel und der Rahmen zusammen gehandhabt werden können.

Der Deckel ist mittels des Rahmens an der Wandung befestigbar ausgebildet. Der Deckel kann mittels des Rahmens mit der Wandung reversibel verbunden bzw. reversibel an der Wandung fixiert werden. Der Rahmen kann als Halterung für den Deckel (Deckelhalterung) ausgebildet sein.

Der Deckel und/oder der Rahmen können autoklavierbar ausgebildet sein. Der Deckel und/oder der Rahmen können autoklavierbares Material umfassen oder aus diesem bestehen. Der Deckel und/oder der Rahmen können derart dimensioniert sein (Größe und/oder Gewicht), dass eine, insbesondere einhändige, Handhabung durch eine Bedienperson möglich ist. Insbesondere kann der Deckel und/oder der Rahmen ein Gewicht von kleiner 5 kg (Kilogramm), vorzugsweise kleiner als 2,5 kg, bevorzugt kleiner als 1,5 kg aufweisen.

Hierdurch kann der Deckel samt dem Rahmen separat dekontaminiert, sterilisiert und/oder autoklaviert werden. Dies kann insbesondere extern, außerhalb der beiden Module, insbesondere außerhalb der pharmazeutischen Anlage, umgesetzt werden. Nach der Dekontamination bzw. dem Autoklavieren kann der Deckel mit dem Rahmen wieder an der Wandung befestigt werden. Anschließend können die beiden Module unabhängig voneinander dekontaminiert werden. Dies kann zeitlich nacheinander, insbesondere in einer beliebigen Reihenfolge, oder gleichzeitig geschehen.

So kann die Dekontamination der pharmazeutischen Anlage wesentlich flexibler gestaltet werden. Es kann bspw. das erste Modul dekontaminiert werden, während das zweite noch gerüstet wird. Die beiden Module können gleichzeitig dekontaminiert werden, sodass die Dekontaminationsphase der gesamten pharmazeutischen Anlage verkürzt werden kann.

Als Dekontaminationsmittel kann Wasserstoffperoxid (H2O2) verwendet werden.

Gemäß einer Weiterbildung kann der Rahmen mittels mindestens einer ersten Schraubverbindung mit der Wandung dicht verbindbar (reversibel) ausgebildet sein. Alternativ oder zusätzlich kann der Rahmen mittels mindestens einer ersten Klemmverbindung mit der Wandung dicht verbindbar (reversibel) ausgebildet sein.

Hierdurch kann die reversible Verbindung des Rahmens mit der Wandung mit einfachen Mitteln umgesetzt werden. Mit anderen Worten, der Rahmen kann so mit einfachen Mitteln dicht bzw. abdichtend mit der Wandung reversibel verbunden werden.

Vorliegend ist mit einer reversiblen Verbindung gemeint, dass zwei Elemente lösbar miteinander verbunden werden können. Mit anderen Worten, eine Verbindung zwischen zwei reversibel miteinander verbundenen Elementen kann gelöst und/oder wiederhergestellt werden.

Vorliegend ist mit "dicht" bzw. "abdichtend" gemeint, dass eine Fluidkommunikation nicht möglich ist. Mit anderen Worten, eine dichte bzw. abgedichtete Verbindung verhindert einen Fluidaustausch zwischen zwei Elementen.

Gemäß einer Weiterbildung kann der Deckel dicht mit dem Rahmen verbindbar und/oder von dem Rahmen lösbar ausgebildet sein. Mit anderen Worten, der Deckel kann dicht reversibel mit dem Rahmen verbindbar ausgebildet sein.

Hierdurch kann eine dichte Verbindung zwischen dem Deckel und dem Rahmen gewährleistet werden. So kann der Deckel in eine (abdichtende) Geschlossenstellung überführt werden, in der die beiden Arbeitsräume fluidisch getrennt sind. Der Deckel kann in eine Offenstellung überführt werden, in der eine Fluidkommunikation zwischen den beiden Arbeitsräumen (der beiden Module) ermöglicht wird.

Gemäß einer Weiterbildung kann der Deckel mittels mindestens einer zweiten Schraubverbindung dicht mit dem Rahmen verbindbar ausgebildet sein. Alternativ oder zusätzlich kann der Deckel mittels mindestens einer zweiten Klemmverbindung dicht mit dem Rahmen verbindbar ausgebildet sein.

Hierdurch kann mit einfachen Mitteln der Deckel mit dem Rahmen dicht und reversibel verbunden werden.

Gemäß einer Weiterbildung kann der Deckel mindestens eine erste Kontaktfläche und der Rahmen mindestens eine zweite Kontaktfläche aufweisen. Der Deckel und der Rahmen können derart ausgebildet sein, dass, wenn der Deckel dicht mit dem Rahmen verbunden ist, die erste Kontaktfläche dicht auf der zweiten Kontaktfläche aufliegt (angeordnet ist). Mit anderen Worten, die erste Kontaktfläche kontaktiert die zweite Kontaktfläche derart, dass der Deckel dichtend mit dem Rahmen verbunden ist. Insbesondere kann die erste Kontaktfläche vollständig auf der zweiten Kontaktfläche aufliegen (und umgekehrt).

Hierdurch kann mit einfachen Mitteln eine abdichtende Verbindung zwischen Deckel und Rahmen umgesetzt werden.

Gemäß einer Weiterbildung kann der Deckel und der Rahmen derart ausgebildet sein, dass, wenn der Deckel von dem Rahmen gelöst (getrennt) ist, die erste Kontaktfläche und die zweite Kontaktfläche jeweils frei liegen. Mit anderen Worten, wenn der Deckel und der Rahmen getrennt sind, sind die erste und die zweite Kontaktfläche voneinander beabstandet angeordnet (kontaktieren einander nicht).

Hierdurch kann gewährleistet werden, dass sowohl die erste Kontaktfläche als auch die zweite Kontaktfläche mit dem Dekontaminationsmittel benetzt bzw. behandelt werden können.

Gemäß einer Weiterbildung kann die erste und/oder die zweite Kontaktfläche jeweils ringförmig oder elliptisch ausgebildet sein. Ebenso sind auch andere geometrische Ausbildungen der ersten und/oder der zweiten Kontaktfläche denkbar.

Hierdurch können die Kontaktflächen minimiert und zeitgleich flexibel ausgestaltet werden. Hierdurch kann Dekontaminationsmittel eingespart und ein mögliches Risiko einer Leckstelle reduziert werden.

Gemäß einer Weiterbildung kann die erste Kontaktfläche, ein erster Querschnitt der ersten Kontaktfläche, die zweite Kontaktfläche und/oder ein zweiter Querschnitt der zweiten Kontaktfläche knickfrei ausgebildet sein. Die erste Kontaktfläche, deren erster Querschnitt, die zweite Kontaktfläche und/oder deren zweiter Querschnitt können jeweils geneigt zur Wandung ausgebildet sein. Mit anderen Worten die Wandung und die erste Kontaktfläche, der erste Querschnitt, die zweite Kontaktfläche und/oder der zweite Querschnitt können jeweils einen Winkel einschließen bzw. aufspannen, der zwischen 0 und 90° liegt, insbesondere 45° beträgt.

Vorliegend ist mit "knickfrei" gemeint, dass es keine Stellen auf der ersten und/oder der zweiten Kontaktfläche gibt, bei welchen sich die Steigung der jeweiligen Fläche sprunghaft ändert. Es gibt insbesondere keine Kanten und/oder Knicke innerhalb der Kontaktflächen bzw. deren jeweiligen Querschnitte. Die beiden Kontaktflächen bzw. deren Querschnitte können zumindest bereichsweise eben ausgebildet sein.

Hierdurch kann ein mögliches Risiko einer Leckstelle, die durch einen Knick in den Kontaktflächen bedingt sein kann, reduziert werden. Die Dichtheit und/oder die Reinigbarkeit zwischen dem Deckel und dem Rahmen kann weiter optimiert werden.

Gemäß einer Weiterbildung kann der Deckel rund oder oval ausgebildet sein. Ebenso denkbar ist es, dass der Deckel eine andere geometrische Form aufweisen kann (bspw. rund, halbrund, rechteckig, quadratisch, dreieckig, vieleckig, etc.).

Hierdurch kann die Form des Deckels flexibel an die entsprechenden Gegebenheiten und Anforderungen angepasst werden.

Der Deckel und der Rahmen sind aus dem ersten Modul oder dem zweiten Modul herausnehmbar ausgebildet. Der Deckel und der Rahmen können aus der pharmazeutischen Anlage herausnehmbar ausgebildet sein. Hierfür können der Deckel und der Rahmen entsprechend dimensioniert (Größe, Form und/oder Gewicht) sein.

Hierdurch können der Deckel und der Rahmen aus dem ersten Modul, dem zweiten Modul und/oder der pharmazeutischen Anlage herausgenommen werden und extern (außerhalb des ersten Moduls, des zweiten Moduls und/oder der pharmazeutischen Anlage) dekontaminiert bzw. autoklaviert werden.

Gemäß einer Weiterbildung kann die Schnittstelle mindestens eine Blende mit einer Durchreichöffnung zum Durchreichen eines Packmittels umfassen. Die Durchreichöffnung kann kleiner ausgebildet sein als die in der Wandung angeordnete Öffnung. Die Blende kann an dem Rahmen angeordnet sein. Ebenso ist es denkbar, dass die Blende an der Wandung angeordnet sein kann. Die Blende kann als Formatteil ausgebildet sein. Die Blende kann fix mit dem Rahmen und/oder mit der Wandung verbunden sein. Ebenso denkbar ist es, dass die Blende wechselbar (reversibel lösbar) mit dem Rahmen und/oder mit der Wandung verbindbar ausgebildet oder verbunden sein kann.

Hierdurch kann, wenn der Deckel in der Offenstellung ist, der Fluidaustausch (Gasaustausch) durch die Öffnung bzw. durch die Durchreichöffnung begrenzt werden. Wenn der Deckel geöffnet wird, kann so der Fluidaustausch, der insbesondere aufgrund unterschiedlicher Drücke innerhalb des ersten und des zweiten Arbeitsraums entstehen kann, begrenzt werden. Der Fluidaustausch kann durch die Wahl der Form bzw. der Dimensionierung der Durchreichöffnung wunschgemäß eingestellt werden.

Die obige Aufgabe wird weiter durch ein Verfahren zum Betreiben einer pharmazeutischen Anlage mit den Merkmalen des Anspruchs 11 gelöst.

Das Verfahren umfasst die Schritte:
Bereitstellen der pharmazeutischen Anlage gemäß obiger Ausführungen.

Lösen des Rahmens mit dem Deckel von der Wandung.

Dekontaminieren, insbesondere Autoklavieren, des Rahmens und des Deckels.

Dies kann außerhalb der Module bzw. der pharmazeutischen Anlage geschehen. Denkbar ist auch, dass die Dekontamination bzw. das Autoklavieren des Deckels und des Rahmens (bspw. in einem Folienbeutel) innerhalb der pharmazeutischen Anlage bzw. innerhalb eines der Module umgesetzt werden kann. Ebenso ist es denkbar, dass die pharmazeutische Anlage eine Dekontaminationskammer umfasst, in der der Rahmen und der Deckel, insbesondere getrennt voneinander, dekontaminiert werden können (die Dekontamination also außerhalb der Module, aber innerhalb der pharmazeutischen Anlage, geschieht).

Hinsichtlich der mit dem Verfahren erzielbaren Vorteile wird auf die diesbezüglichen Ausführungen zur pharmazeutischen Anlage verwiesen. Zur weiteren Ausgestaltung des Verfahrens können die im Zusammenhang mit der pharmazeutischen Anlage beschriebenen und/oder die nachfolgend noch erläuterten Maßnahmen dienen.

Gemäß einer Weiterbildung kann das Verfahren die Schritte umfassen:
Lösen des Deckels von dem Rahmen vor der Dekontamination, sodass die erste Kontaktfläche und die zweite Kontaktfläche frei liegen und für ein Dekontaminationsmittel zugänglich sind.

Verbinden des Deckels mit dem Rahmen nach der Dekontamination, sodass die erste Kontaktfläche und die zweite Kontaktfläche dichtend aufeinanderliegen.

Hierdurch kann gewährleistet werden, dass die erste und die zweite Kontaktfläche vollständig mittels des Dekontaminationsmittels benetzt werden. Sobald die erste und die zweite Kontaktfläche dichtend aufeinanderliegen, kann keine Kontamination der Kontaktflächen mehr stattfinden. Aufgrund der Dichtheit zwischen den beiden Kontaktflächen ist dies schlicht nicht möglich.

Gemäß einer Weiterbildung kann das Verfahren die Schritte umfassen:
Platzieren des Rahmens und des Deckels in einem Folienbeutel.

Dekontaminieren, insbesondere Autoklavieren, des Rahmens und des Deckels in dem Folienbeutel.

Der Schritt des Verbindens des Deckels mit dem Rahmen kann nach der Dekontamination in dem Folienbeutel durchgeführt werden.

Der Deckel und der Rahmen können in einem voneinander gelösten Zustand in dem Folienbeutel dekontaminiert bzw. autoklaviert werden. Der Deckel und der Rahmen können nach der Dekontamination bzw. nach dem Autoklavieren im Folienbeutel (wieder) miteinander dichtend verbunden werden.

So kann der Deckel mit dem Rahmen in der noch sterilen Umgebung des Folienbeutels verbunden werden. Eine Kontamination, insbesondere der Kontaktflächen, wird so vermieden.

Anschließend kann der Rahmen samt dem Deckel an der Wandung angebracht bzw. angeordnet werden. Dabei bleiben insbesondere die Kontaktflächen zwischen dem Rahmen und dem Deckel steril.

Gemäß einer Weiterbildung kann das Verfahren die Schritte umfassen:
Herausnehmen des Rahmens und des Deckels aus dem ersten Modul und/oder dem zweiten Modul, insbesondere aus der pharmazeutischen Anlage.

Dekontaminieren, insbesondere Autoklavieren des Rahmens und des Deckels außerhalb des ersten Moduls und/oder des zweiten Moduls, insbesondere außerhalb der pharmazeutischen Anlage.

Hierdurch kann das Dekontaminieren bzw. Autoklavieren des Rahmens und des Deckels extern (von dem ersten Modul, dem zweiten Modul und/oder der pharmazeutischen Anlage entkoppelt) umgesetzt werden. Hierdurch kann die Flexibilität der Dekontamination der gesamten pharmazeutischen Anlage erhöht werden.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Darstellung einer pharmazeutischen Anlage mit zwei Modulen und einer Schnittstelle;
- Fig. 2: eine schematische Draufsicht auf die Schnittstelle gemäß Figur 1 mit einem Rahmen und einem Deckel;
- Fig. 3: eine schematische Schnittansicht der Schnittstelle gemäß dem in Figur 2 gezeigten Schnitt A-A;
- Fig. 4: einen schematische Schnittansicht der Schnittstelle gemäß Figur 3 gemäß einem zweiten Ausführungsbespiel;
- Fig. 5: einen Ausschnitt einer Schnittansicht des Rahmens und des Deckels gemäß einem dritten Ausführungsbeispiel und
- Fig. 6: eine schematische Schnittansicht des Rahmens und des Deckels gemäß Figur 2 während einer Dekontamination bzw. eines Autoklavierens.

In der nachfolgenden Beschreibung sowie in den Figuren tragen sich entsprechende Bauteile und Elemente gleiche Bezugszeichen. Der besseren Übersichtlichkeit wegen sind nicht in allen Figuren sämtliche Bezugszeichen wiedergegeben.

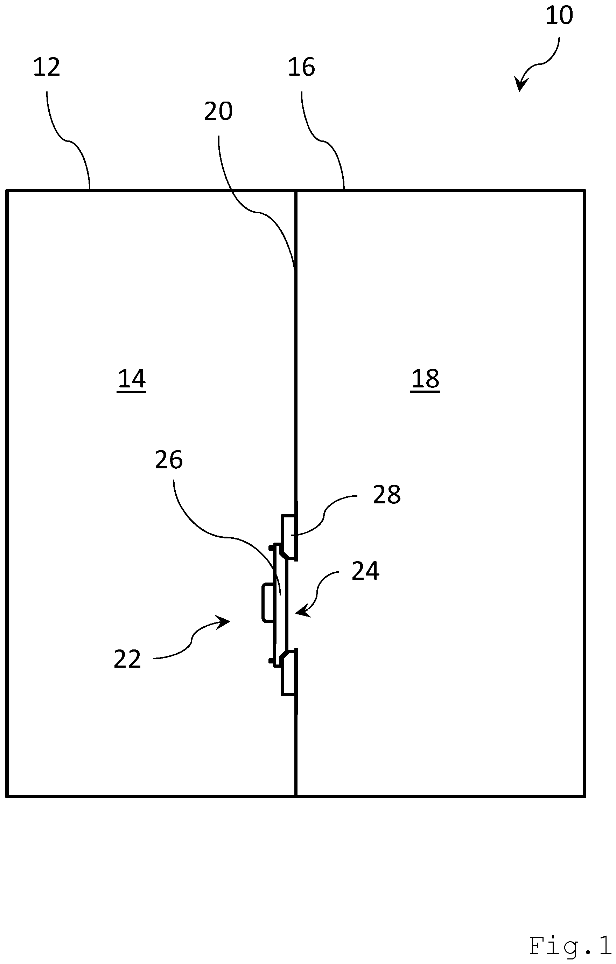

Figur 1 zeigt eine schematische Darstellung einer pharmazeutischen Anlage 10. Die pharmazeutische Anlage 10 ist vorliegend zum aseptischen Abfüllen von Pharmazeutika eingerichtet.

Die pharmazeutische Anlage 10 umfasst ein erstes Modul 12 mit einem gegenüber seiner Umgebung abgeschlossenen ersten Arbeitsraum 14. Die pharmazeutische Anlage 10 umfasst ein zweites Modul 16 mit einem gegenüber seiner Umgebung abgeschlossenen zweiten Arbeitsraum 18. Die pharmazeutische Anlage 10 umfasst vorliegend eine Wandung 20, die die beiden Arbeitsräume 14, 18 räumlich getrennt.

Die pharmazeutische Anlage 10 umfasst eine Schnittstelle 22. Die Schnittstelle 22 umfasst eine in der Wandung 20 angeordnete Öffnung 24. Mittels der Öffnung 24 können die beiden Arbeitsräume 14, 18 fluidisch miteinander verbunden bzw. gekoppelt werden.

Die Schnittstelle 22 umfasst einen Deckel 26 zum Verschließen und Freigeben der Öffnung 24. Der Deckel 26 gibt in einer Offenstellung die Öffnung 24 frei und verschließt die Öffnung 24 in einer Geschlossenstellung. Der Deckel 26 kann aus der Offenstellung in die Geschlossenstellung überführt werden (und umgekehrt). Insbesondere verschließt der Deckel 26 die Öffnung 24 in der Geschlossenstellung dicht bzw. abdichtend. Mit anderen Worten, in der Geschlossenstellung verhindert der Deckel 26 eine Fluidkommunikation zwischen den beiden Arbeitsräumen 14, 18 durch die Öffnung 24 (die beiden Arbeitsräume 14, 18 sind fluidisch getrennt). In der Offenstellung gibt der Deckel 26 die Öffnung 24 frei, sodass die beiden Arbeitsräume 14, 18 in einer Fluidkommunikation stehen (die beiden Arbeitsräume 14, 18 sind fluidisch verbunden).

Die Schnittstelle 22 umfasst einen lösbar mit der Wandung 20 verbindbaren Rahmen 28. Der Rahmen kann dicht bzw. abdichtend an der Wandung 20 angebracht bzw. mit der Wandung 20 verbunden werden. Der Rahmen 28 ist insbesondere an den die Wandung 20 kontaktierenden Flächen dicht mit der Wandung 20 verbunden.

Der Deckel 26 kann mittels des Rahmens 28 an der Wandung 20 befestigt werden. Mit anderen Worten, der Deckel 26 ist mittels des Rahmens 28 mit der Wandung reversibel (und abdichtend) verbindbar ausgebildet.

Der Deckel 26 und der Rahmen 28 sind vorliegend derart ausgebildet, dass diese aus dem ersten Modul, dem zweiten Modul und/oder der pharmazeutischen Anlage 10 herausgenommen werden können.

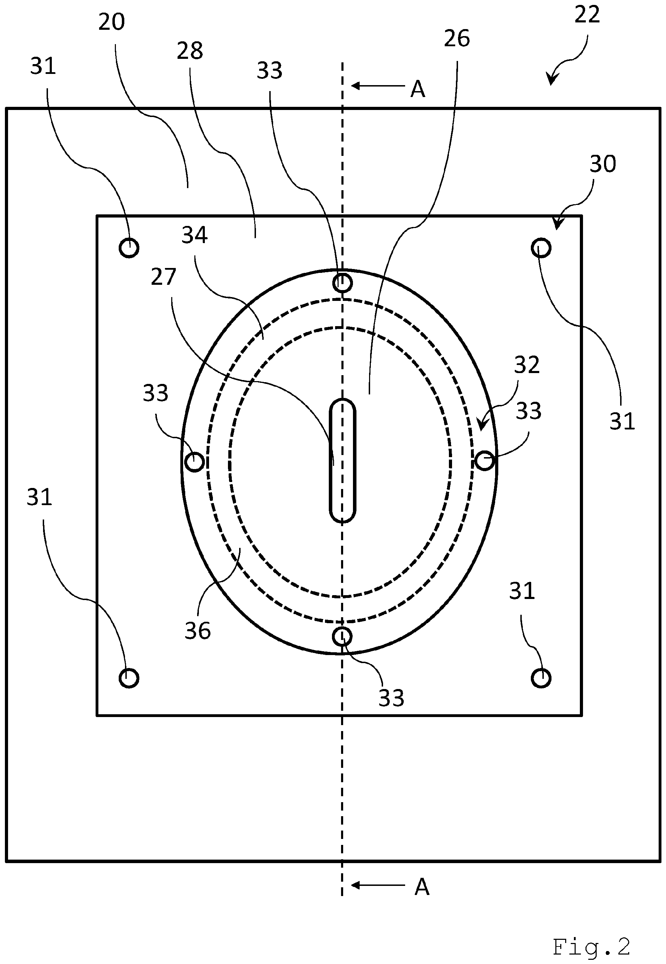

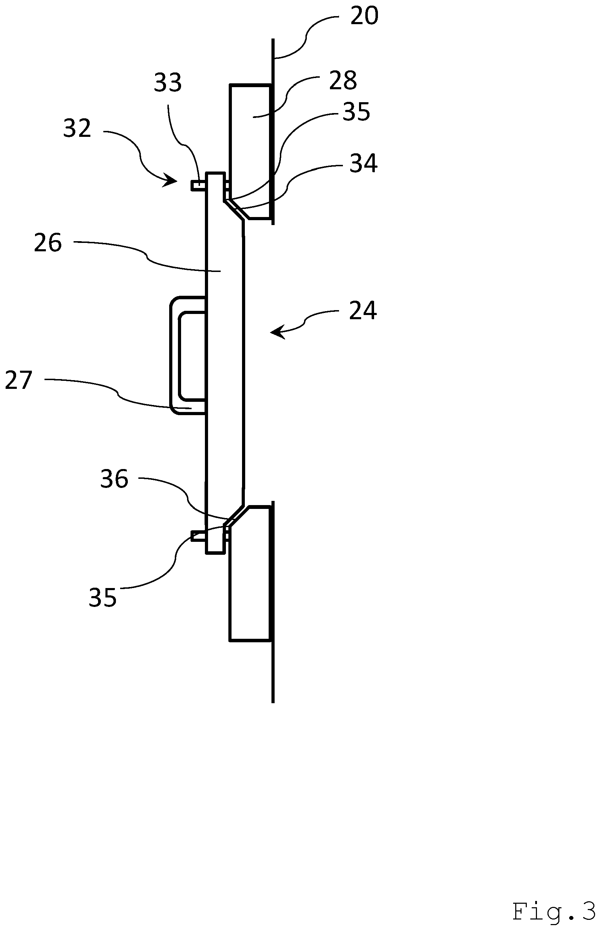

Figur 2 zeigt eine schematische Draufsicht auf die Schnittstelle 22 gemäß Figur 1 und Figur 3 zeigt eine schematische Schnittansicht der Schnittstelle 22 gemäß dem in Figur 2 gezeigten Schnitt A-A.

Der Rahmen 28 ist vorliegend rechteckig, insbesondere quadratisch, ausgebildet. Der Rahmen 28 ist vorliegend mittels einer ersten Schraubverbindung 30 mit der Wandung 20 dicht verbindbar ausgebildet. Die erste Schraubverbindung 30 umfasst vorliegend vier Schrauben 31, die jeweils in einer Ecke des rechteckigen Rahmens 28 angeordnet sind.

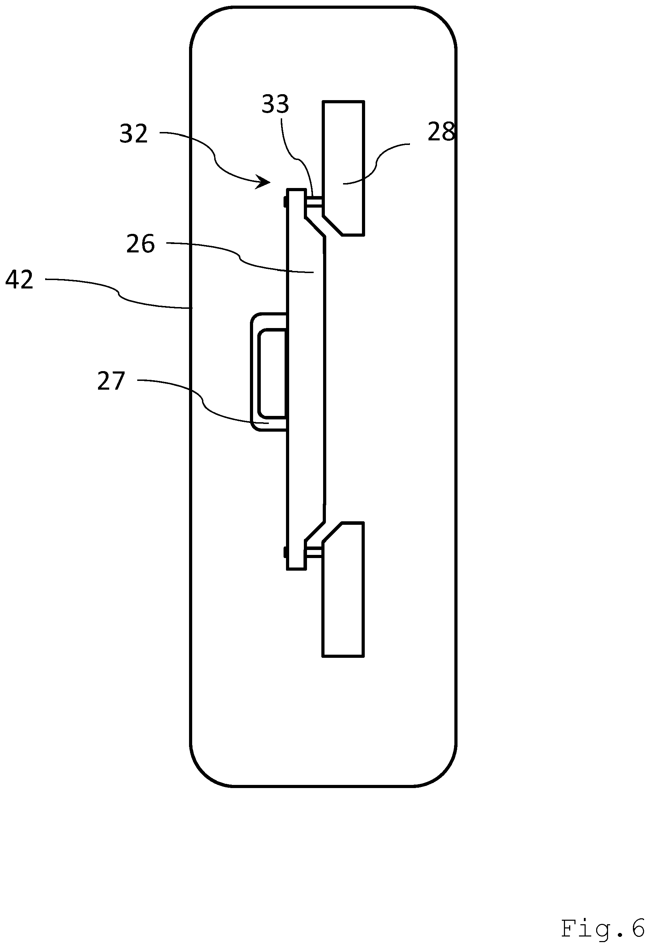

Der Deckel 26 ist dicht mit dem Rahmen 28 verbindbar (vgl. Figur 2 bzw. Figur 3) und/oder von dem Rahmen 28 lösbar ausgebildet (vgl. Figur 6). Der Deckel 26 ist vorliegend oval ausgebildet.

Der Deckel 26 ist vorliegend mittels einer zweiten Schraubverbindung 32 dicht mit dem Rahmen 28 verbindbar ausgebildet. Die zweite Schraubverbindung 32 umfasst vorliegend vier Schrauben 33, die paarweise, einander gegenüberliegend angeordnet sind.

Es ist denkbar, dass die Schrauben 33 als Bolzen ausgebildet sein können, die den Deckel 26 und den Rahmen 28 in einem definierten Abstand zueinander halten können. Die Bolzen können derart ausgebildet sein, dass der Deckel 26 in mehreren definierten Abständen von dem Rahmen 28 jeweils arretierbar ist. So kann der Abstand zwischen dem Deckel 26 und dem Rahmen 28 kontrolliert vergrößert werden. Hierdurch kann ein ruckartiges Öffnen des Deckels 26 verhindert werden. Damit kann der Fluidaustausch, insbesondere beim Öffnen des Deckels 26, durch die Schnittstelle 22 bzw. durch die Öffnung 24 kontrolliert eingestellt werden. Der Deckel 26 kann mittels der Bolzen geführt werden. Mit anderen Worten, die Bolzen können eine Führung für den Deckel 26 während des Trennens des Deckels 26 von dem Rahmen 28 bzw. während des Verbindens des Deckels 26 mit dem Rahmen 28 darstellen.

Der Deckel 26 umfasst vorliegend einen Griff 27. Der Deckel kann mittels des Griffs 27 gehandhabt werden. Der Deckel 26 kann bspw. mittels des Griffs 27 aus der Geschlossenstellung in die Offenstellung (und umgekehrt) überführt werden. Mittels des Griffs 27 kann der Deckel 26 samt dem Rahmen 28 (insbesondere wenn der Deckel 26 mit dem Rahmen 28 verbunden ist) gehandhabt werden.

Der Deckel 26 umfasst vorliegend eine erste Kontaktfläche 34 und der Rahmen 28 eine zweite Kontaktfläche 36. Wenn der Deckel 26 dicht mit dem Rahmen 28 verbunden ist, liegen die erste Kontaktfläche 34 und die zweite Kontaktfläche 36 dicht bzw. abdichtend aufeinander.

Vorliegend sind die erste Kontaktfläche 34 und die zweite Kontaktfläche 36 jeweils elliptisch ausgebildet (vgl. Figur 2). Die erste und die zweite Kontaktfläche 34, 36 weisen vorliegend einen Knick 35 auf (vgl. Figur 3). Die erste und die zweite Kontaktfläche 34, 36 weisen somit jeweils zwei knickfreie Teilflächen auf.

Der besseren Übersicht wegen, sind die Kontaktflächen 34, 36 in Figur 3 beabstandet dargestellt. Wenn die Kontaktflächen 34, 36 dichtend aufeinander aufliegen, ist kein Abstand zwischen den Kontaktflächen 34, 36 vorhanden.

Wenn der Deckel 26 von dem Rahmen 28 gelöst ist, liegen die beiden Kontaktflächen 34, 36 jeweils frei (vgl. Figur 6), sodass ein Dekontaminationsmittel die Kontaktflächen 34, 36 vollständig benetzen kann.

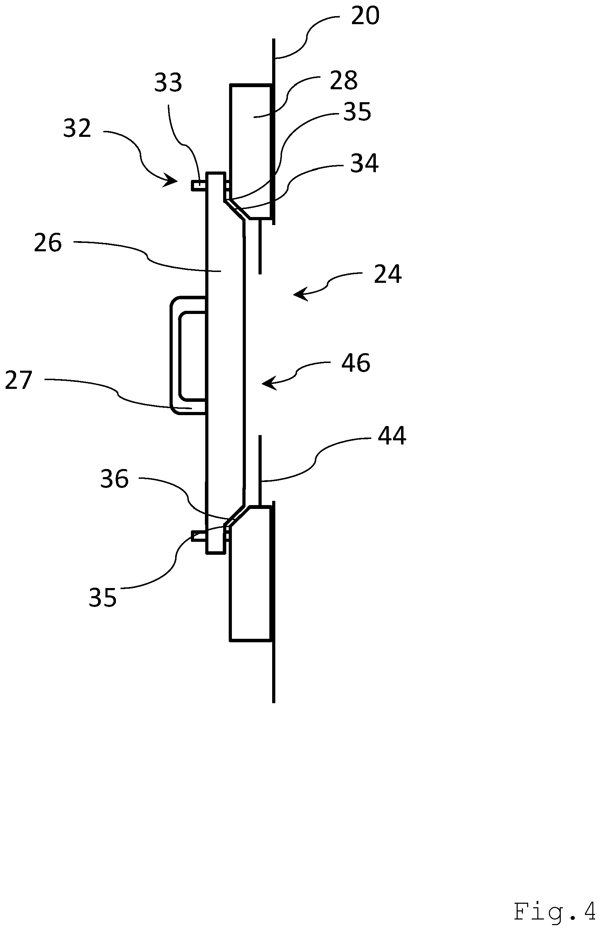

Figur 4 zeigt schematische Schnittansicht der Schnittstelle 22 gemäß Figur 3 gemäß einem zweiten Ausführungsbeispiel. Das in Figur 4 gezeigte Ausführungsbeispiel unterscheidet sich von dem in den Figuren 1 bis 3 gezeigten Ausführungsbeispiel durch Folgendes:
Die Schnittstelle 22 umfasst vorliegend eine Blende 44. Die Blende 44 weist eine Durchreichöffnung 46 auf. Die Blende 44 dient zum Durchreichen eines Packmittels (in den das Pharmazeutika abgefüllt werden kann) aus dem ersten Modul 12 bzw. dessen Arbeitsraum 14 in das zweite Modul 16 bzw. dessen Arbeitsraum 18 (bzw. umgekehrt).

Die Durchreichöffnung 46 ist kleiner ausgebildet als die Öffnung 24. Die Durchreichöffnung 46 kann die Form eines Packmittels entsprechen. Mit anderen Worten, die Durchreichöffnung 46 kann die Kontur eines Packmittels aufweisen.

Die Blende 44 ist vorliegend an dem Rahmen 28 (zwischen dem Deckel 26 und der Öffnung 24) angeordnet. Die Blende 44 kann fix an dem Rahmen 28 angeordnet sein. Die Blende 44 kann ebenso als Formatteil (wechselbar) ausgebildet sein. Die Blende 44 kann abhängig von den verwendeten Packmitteln gewählt werden.

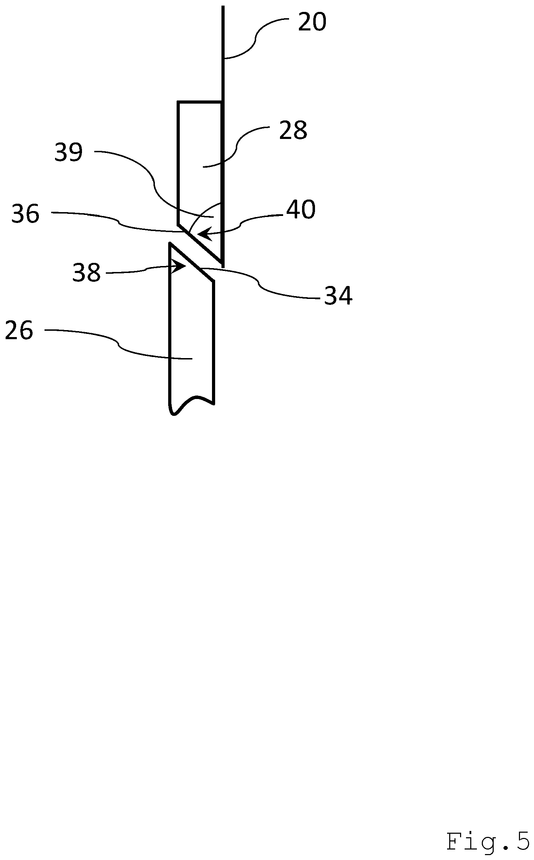

Figur 5 zeigt einen Ausschnitt einer Schnittansicht des Rahmens 28 und des Deckels 26 gemäß einem dritten Ausführungsbeispiel. Das in Figur 5 gezeigte Ausführungsbeispiel unterscheidet sich von dem in den Figuren 1 bis 3 gezeigten Ausführungsbeispiel durch die anders ausgebildeten Kontaktflächen 34, 36.

Die Kontaktflächen 34, 36 sind vorliegend knickfrei ausgebildet. Die Kontaktflächen 34, 36 weisen jeweils eine Kegelsegment-artige Form auf. Mit anderen Worten, die Kontaktflächen 34, 36 entsprechen jeweils einem Ausschnitt einer Mantelfläche eines Kegels bzw. einem Kegelstumpf.

Vorliegend ist ein erster Querschnitt 38 der ersten Kontaktfläche 34 und ein zweiter Querschnitt 40 der zweiten Kontaktfläche 36 ebenfalls knickfrei ausgebildet. Vorliegend sind die beiden Querschnitte 38, 40 der Kontaktflächen 34, 36 geneigt zur Wandung 20 orientiert. Vorliegend ist jeweils zwischen der ersten Kontaktfläche 34, dem ersten Querschnitt 38, der zweiten Kontaktfläche 36, dem zweiten Querschnitt 40 und der Wandung 20 ein Winkel 39 vorhanden, der vorliegend 45° beträgt. Mit anderen Worten, der erste Querschnitt 38 (bzw. die erste Kontaktfläche 34) und der zweite Querschnitt 40 (bzw. die zweite Kontaktfläche 36) sind vorliegend 45° geneigt zur Wandung 20 orientiert ausgebildet.

Im Folgenden wird anhand der Figur 6 ein Verfahren zum Betreiben der pharmazeutischen Anlage 10 erläutert. Figur 6 zeigt eine schematische Schnittansicht des Rahmens 28 und des Deckels 26 gemäß Figur 2 während einer Dekontamination bzw. während des Autoklavierens. Im Folgenden wird insbesondere das Dekontaminieren der pharmazeutischen Anlage 10 beschrieben.

Nach dem Bereitstellen der pharmazeutischen Anlage 10 wird zunächst der Rahmen 28 mit dem Deckel 26 von der Wandung 20 gelöst. Anschließend wird der Rahmen 28 und der Deckel 26 dekontaminiert, insbesondere autoklaviert.

Vor der Dekontamination bzw. vor dem Autoklavieren wird der Deckel 26 von dem Rahmen 28 gelöst bzw. getrennt, sodass die erste Kontaktfläche 34 und die zweite Kontaktfläche 36 freiliegen und für ein Dekontaminationsmittel zugänglich sind. Das Dekontaminationsmittel kann so beide Kontaktflächen 34, 36 vollständig benetzen.

Nach dem Dekontaminieren bzw. Autoklavieren wird der Deckel 26 mit dem Rahmen 28 verbunden, sodass die erste Kontaktfläche 34 und die zweite Kontaktfläche 36 dichtend aufeinanderliegen. Solange die beiden Kontaktflächen 34, 36 dichtend aufeinanderliegen, kann eine Kontamination insbesondere der Kontaktflächen 34, 36 ausgeschlossen werden. Dies wird aufgrund der Dichtheit zwischen den Kontaktflächen 34, 36 vermieden. Für eine Kontamination der Kontaktflächen 34, 36 müssten diese zumindest teilweise freiliegen.

Für die Dekontamination bzw. das Autoklavieren des Deckels 26 und des Rahmens 28 werden diese in einen Folienbeutel 42 platziert (vgl. Figur 6). Der Rahmen 28 und der Deckel 26 werden innerhalb des Folienbeutels 42 dekontaminiert bzw. autoklaviert. Nach dem Dekontaminieren bzw. Autoklavieren werden der Deckel 26 und der Rahmens 28 noch im Folienbeutel 42 wieder miteinander verbunden. Dies kann bspw. durch ein Aufeinanderdrücken (bzw. -pressen) des Deckels 26 auf den Rahmen 28, bspw. mittels des Griffs 27, umgesetzt werden. Hierzu kann eine Kraft bspw. durch eine Bedienperson von außerhalb des Folienbeutels 42 auf den Deckel 26 und/oder den Rahmen 28 (durch den Folienbeutel 42 hindurch) ausgeübt werden, sodass die Kontaktflächen 34, 36 innerhalb des Folienbeutels 42 (dichtend) aufeinandergelegt werden.

Anschließend können der Deckel 26 und der Rahmen 28 in dem miteinander verbundenen Zustand aus dem Folienbeutel 42 herausgenommen und an der Wandung 20 angeordnet (fixiert) werden. Dabei werden die Kontaktflächen 34, 36 nicht freigelegt, bleiben also dekontaminiert bzw. steril.

Nach dem Fixieren des Rahmens 28 samt dem Deckel 26 an der Wandung 20 können die beiden Module 12, 16 bzw. deren Arbeitsräume 14, 18 dekontaminiert werden. Dies kann nacheinander oder zeitgleich umgesetzt werden. In beiden Fällen wird die Schnittstelle 22 insbesondere aufgrund der sterilen Kontaktflächen 34, 36 optimal dekontaminiert.

Das Dekontaminieren bzw. Autoklavieren des Rahmens 28 und des Deckels 26 kann außerhalb des ersten Moduls 12, des zweiten Moduls 16 und/oder außerhalb der pharmazeutischen Anlage 10 geschehen. Hierzu kann der Deckel 26 und der Rahmen 28 zunächst aus dem ersten Modul 12, dem zweiten Modul 16 und/oder aus der pharmazeutischen Anlage 10 herausgenommen werden. Anschließend kann der Deckel 26 und der Rahmen 28 bspw. in dem Folienbeutel 42 (extern) dekontaminiert bzw. autoklaviert werden.

Vor dem Dekontaminieren bzw. Autoklavieren können der Rahmen 28 und der Deckel 26 voneinander getrennt werden, sodass die Kontaktflächen 34, 36 freiliegen. Dies kann im Folienbeutel 42 oder bereits vorher, vor dem Platzieren des Rahmens 28 und des Deckels 26 in den Folienbeutel 42, geschehen.

## Patentansprüche

1. Pharmazeutische Anlage (10) zum, insbesondere aseptischen, Abfüllen von Pharmazeutika umfassend:
- ein erstes Modul (12) mit einem gegenüber seiner Umgebung abgeschlossenen ersten Arbeitsraum (14),
- ein zweites Modul (16) mit einem gegenüber seiner Umgebung abgeschlossenen zweiten Arbeitsraum (18),
- mindestens eine Wandung (20) zur räumlichen Trennung der Arbeitsräume (14, 18),
- mindestens eine Schnittstelle (22) mit mindestens einer in der Wandung (20) angeordneten Öffnung (24) zum Verbinden des ersten Arbeitsraums (14) mit dem zweiten Arbeitsraum (18), wobei die Schnittstelle (22) einen Deckel (26) zum Verschließen und Freigeben der Öffnung (24) umfasst, wobei der Deckel (26) in einer Offenstellung die Öffnung (24) freigibt und in einer Geschlossenstellung die Öffnung (24) verschließt, **dadurch gekennzeichnet, dass**
die Schnittstelle (22) einen lösbar mit der Wandung (20) verbindbaren Rahmen (28) umfasst, wobei der Deckel (26) mittels des Rahmens (28) an der Wandung (20) befestigbar ausgebildet ist, wobei der Deckel (26) und der Rahmen (28) aus dem ersten Modul (12) oder dem zweiten Modul (16), insbesondere aus der pharmazeutischen Anlage (10), herausnehmbar ausgebildet sind.

2. Pharmazeutische Anlage nach Anspruch 1, **dadurch gekennzeichnet, dass** der Rahmen (28) mittels mindestens einer ersten Schraubverbindung (30) und/oder einer ersten Klemmverbindung mit der Wandung (20) dicht verbindbar ausgebildet ist.

3. Pharmazeutische Anlage nach einem der voranstehenden Ansprüche, **dadurch gekennzeichnet, dass** der Deckel (26) dicht mit dem Rahmen (28) verbindbar und/oder von dem Rahmen (28) lösbar ausgebildet ist.

4. Pharmazeutische Anlage nach Anspruch 3, **dadurch gekennzeichnet, dass** der Deckel (26) mittels mindestens einer zweiten Schraubverbindung (32) und/oder einer zweiten Klemmverbindung dicht mit dem Rahmen (28) verbindbar ausgebildet ist.

5. Pharmazeutische Anlage nach einem der voranstehenden Ansprüche, **dadurch gekennzeichnet, dass** der Deckel (26) mindestens eine erste Kontaktfläche (34) und der Rahmen (28) mindestens eine zweite Kontaktfläche (36) aufweisen, wobei der Deckel (26) und der Rahmen (28) derart ausgebildet sind, dass, wenn der Deckel (26) dicht mit dem Rahmen (28) verbunden ist, die erste Kontaktfläche (34) dicht auf der zweiten Kontaktfläche (36) aufliegt.

6. Pharmazeutische Anlage (10) nach Anspruch 5, **dadurch gekennzeichnet, dass** der Deckel (26) und der Rahmen (28) derart ausgebildet sind, dass, wenn der Deckel (26) von dem Rahmen (28) gelöst ist, die erste Kontaktfläche (34) und die zweite Kontaktfläche (36) jeweils frei liegen.

7. Pharmazeutische Anlage (10) nach Anspruch 5 oder 6, **dadurch gekennzeichnet, dass** die erste und/oder die zweite Kontaktfläche (34, 36) jeweils ringförmig oder elliptisch ausgebildet sind.

8. Pharmazeutische Anlage (10) nach einem der Ansprüche 5 bis 7, **dadurch gekennzeichnet, dass** die erste Kontaktfläche (34), ein erster Querschnitt (38) der ersten Kontaktfläche (34), die zweite Kontaktfläche (38) und/oder ein zweiter Querschnitt (40) der zweiten Kontaktfläche (36) knickfrei, insbesondere geneigt zur Wandung (20), ausgebildet sind.

9. Pharmazeutische Anlage (10) nach einem der voranstehenden Ansprüche, **dadurch gekennzeichnet, dass** der Deckel (26) rund oder oval ausgebildet ist.

10. Pharmazeutische Anlage (10) nach einem der voranstehenden Ansprüche, dass die Schnittstelle (22) mindestens eine Blende (44) mit einer Durchreichöffnung (46) zum Durchreichen eines Packmittels umfasst, wobei die Durchreichöffnung (46) kleiner ausgebildet ist als die Öffnung (24), insbesondere wobei die Blende (44) an dem Rahmen (28) angeordnet ist.

11. Verfahren zum Betreiben einer pharmazeutischen Anlage (10) umfassend die Schritte:
- Bereitstellen der pharmazeutischen Anlage (10) nach einem der voranstehenden Ansprüche;
- Lösen des Rahmens (28) mit dem Deckel (26) von der Wandung (20);
- Dekontaminieren, insbesondere Autoklavieren, des Rahmens (28) und des Deckels (26).

12. Verfahren nach Anspruch 11, **gekennzeichnet durch** die Schritte:
- Lösen des Deckels (26) von dem Rahmen (28) vor der Dekontamination, so dass die erste Kontaktfläche (34) und die zweite Kontaktfläche (36) freiliegen und für ein Dekontaminationsmittel zugänglich sind;
- Verbinden des Deckels (26) mit dem Rahmen (28) nach der Dekontamination, so dass die erste Kontaktfläche (34) und die zweite Kontaktfläche (36) dichtend aufeinander liegen.

13. Verfahren nach Anspruch 12, **gekennzeichnet durch** die Schritte:
- Platzieren des Rahmens (28) und des Deckels (26) in einen Folienbeutel (42);
- Dekontaminieren, insbesondere Autoklavieren, des Rahmens (28) und des Deckels (26) in dem Folienbeutel (42),
- wobei der Schritt des Verbindens des Deckels (26) mit dem Rahmen (28) nach der Dekontamination in dem Folienbeutel (42) durchgeführt wird.

14. Verfahren nach einem der Ansprüche 11 bis 13, **gekennzeichnet durch** die Schritte:
- Herausnehmen des Rahmens (28) und des Deckels (26) aus dem ersten Modul (12), dem zweiten Modul (16) und/oder aus der pharmazeutischen Anlage (10);
- Dekontaminieren, insbesondere Autoklavieren, des Rahmens (28) und des Deckels (26) außerhalb des ersten Moduls (12), des zweiten Moduls (16) und/oder außerhalb der pharmazeutischen Anlage (10).
